# EUROPEAN PATENT SPECIFICATION

(11) **EP 1 897 539 B1**
(45) Date of publication and mention of the grant of the patent: **31.10.2012**
(21) Application number: 06767074.5
(22) Date of filing: 21.06.2006
(51) Int. Cl.: A61K 31/09, A61K 31/122, A61K 31/385, A61K 31/19, A61K 45/06, A61P 3/02, A61P 43/00

(54) **ANTI-FATIGUE COMPOSITION**
ZUSAMMENSETZUNG GEGEN MÜDIGKEIT
COMPOSITION ANTI-FATIGUE

(30) Priority: 24.06.2005 JP 2005184463
(43) Date of publication of application: 12.03.2008
(73) Proprietor: Kaneka Corporation, Osaka-shi, Osaka 530-8288 (JP)
(72) Inventor: KISHIDA, Hideyuki, 6750039 (JP); KAWABE, Taizo, 6728044 (JP); HOSOE, Kazunori, 6760025 (JP)
(74) Representative: Gillet, Raphaëlle
(86) International application number: PCT/JP2006/312415
(87) International publication number: WO 2006/137441

(56) References cited:
- EP-A- 1 728 506
- WO-A-2006/060548
- WO-A1-01/21208
- WO-A1-01/52822

## Description

### Technical Field

The present invention relates to an anti-fatigue composition which is a combination product consisting of a reduced coenzyme Q represented by the following formula (1): 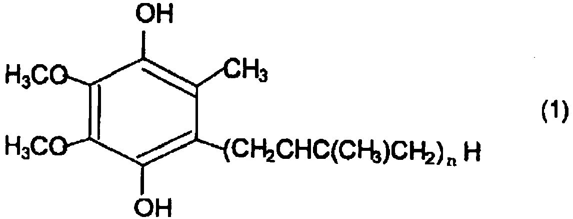 wherein n is an integer of 1 to 12, and
lipoic acid, dihydrolipoic acid, lipoamide, lipoyllysin, di-6,8-bisnorlipoic acid, or tetranorlipoic acid, or a pharmaceutically acceptable salt thereof,
wherein the weight ratio of (a) to (b) is within the range of 10:1 to 1:10.

### Background Art

Heretofore, a number of substances exhibiting an effect of alleviating fatigue have been reported. Lipoic acid and derivatives thereof are among such substances, and so are oxidized coenzyme Q and reduced coenzyme Q.

Coenzyme Q is an essential component widely distributed in living bodies, from bacteria to mammals. In the case of human, coenzyme Q10 in which the side chain thereof has 10 repeating units is known to be the main component. Coenzyme Q is a physiological component existing as a constituent of the electron transport system of mitochondria in cells of living bodies and, through repeated oxidation and reduction in living bodies, functions as a transmitter in the electron transport system.

Coenzyme Q is known to show energy production activity, membrane stabilizing activity and antioxidation activity in living bodies, and thus can be used for wide applications. Of the coenzymes Q, oxidized coenzyme Q (also known as ubiquinone or ubidecarenone) is known to act effectively on the heart and is used for medical purposes as a congestive heart failure drug.

Reported effects of oxidized coenzyme Q include improvement of the oxygen utilization efficiency in the cardiac muscle, activation of ATP production in the cardiac muscle and improvement of cardiac function. In addition to such medical applications, the effects as nutrients and nutritional supplements like those of vitamins have been reported.

JP-A-62-59208 (Patent Document 1) discloses a tissue metabolism-activating composition which is composed of a mixture of ubiquinone and a dry yeast powder, JP-A-52-99220 (Patent Document 2) discloses improvement of symptoms of myasthenia gravis, and JP-A-52-99222 (Patent Document 3) also reports an increase in erythrocytes and the like. Further, effects of recovery from fatigue have been reported in JP-A-7-330584 (patent Document 4), JP-A-7-330593 (Patent Document 5), JP-A-10-287560 (Patent Document 6), and WO2004/066988 (Patent Document 7).

Lipoic acid is a compound having a disulfide bond and is also called thioctic acid. At the time of discovery, lipoic acid was considered as one kind of vitamin. However, it was later found that lipoic acid was bio-synthesized in animal bodies. Lipoic acid has a role of producing energy by metabolizing the nutrients of the ingested food and a role of an antioxidant in the living body. As a pharmaceutical product, lipoic acid in the form of thioctic acid or thioctic acid amide is applied "when the demand for thioctic acid increased due to severe physical fatigue" in Japan, thus attracting attention as an anti-fatigue substance. Moreover, lipoic acid has been used overseas as a therapeutic agent for diabetes.

A combination of lipoic acid and oxidized coenzyme Q is reported to be useful for improving amnesia (Patent Document 8, JP-A-2003-513039). Moreover, it has been reported that a combination of carnitine and lipoic acid with oxidized coenzyme Q as necessary enhances energy and stamina (Patent Document 9, WO2001/21208).
Patent Document 1: JP-A-62-59208
Patent Document 2: JP-A-52-99220
Patent Document 3: JP-A-52-99222
Patent Document 4: JP-A-7-330584
Patent Document 5: JP-A-7-330593
Patent Document 6: JP-A-10-287560
Patent Document 7: WO2004/066988
Patent Document 8: JP-A-2003-513039
Patent Document 9: W02001/21208

### Disclosure of the Invention

### Problems to be Solved by the Invention

The present invention aims at providing a safe anti-fatigue composition, which is effective even at low doses.

### Means of Solving the Problems

As described above, it is known that a mixture of oxidized coenzyme Q, carnitine and lipoic acid is used as an anti-fatigue agent. However, it is not known that a combination of reduced coenzyme Q and lipoic acid alone is effective for prevention of or recovery from fatigue. The present inventors have conducted intensive studies and found that reduced coenzyme Q and lipoic acid exhibit a synergistic effect and that a combination thereof at low doses, at which they are ineffective when used singly, also expresses an anti-fatigue effect, which resulted in the completion of the invention.

Accordingly, the present invention provides the following.
[1] An anti-fatigue combination product consisting of
   (a) reduced coenzyme Q represented by the following formula (1) : 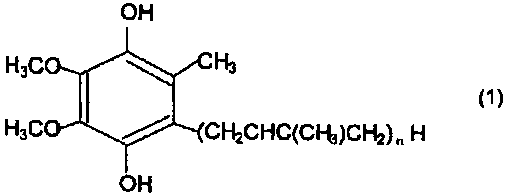 wherein n is an integer of 1 to 12,
   (b) lipoic acid, dihydrolipoic acid, lipoamide, lipoyllysin, di-6,8-bisnorlipoic acid, or tetranorlipoic acid, or a pharmaceutically acceptable salt thereof,
      wherein the weight ratio of (a) to (b) is within the range of 10:1 to 1:10,
   (c) optionally at least one antioxidant selected from the group consisting of vitamin C, probucol, lycopene, vitamin A, carotenoids, vitamin B, flavonoids, polyphenols, glutathione, selenium, sodium thiosulfate, vitamin E, pycnogenol, flavangenol, pyrroloquinoline quinone, superoxide dismutase (SOD), glutathione peroxidase, glutathione-S-transferase, glutathione reductase, catalase and ascorbate peroxidase; and
      optionally one or more carriers chosen from the following groups:
   (d) oils consisting of natural oil, oily higher fatty acid, higher fatty acid monoglyceride, medium-chain triglyceride (MCT)or a mixture thereof,
   (e) excipients consisting of saccharose, lactose, glucose, cornstarch, mannitol, crystalline cellulose, calcium phosphate, calcium sulfate,
   (f) disintegrants consisting of starch, agar, calcium citrate, calcium carbonate, sodium hydrogen carbonate, dextrin, crystalline cellulose, carboxymethyl cellulose, tragacanth,
   (g) lubricants consisting of talc, magnesium stearate, polyethylene glycol, silica, hydrogenated vegetable oil,
   (h) binders consisting of ethyl cellulose, methyl cellulose, hydroxypropylmethyl cellulose, tragacanth, shellac, gelatin; gum arabic, polyvinylpyrrolidone, polyvinyl alcohol, polyacrylic acid, polymethacrylic acid, sorbitol,
   (i) colorants,
   (j) anti-agglomeration agents consisting of stearic acid, talc, light anhydrous silicic acid, hydrous silicon dioxide,
   (k) absorption promoters consisting of surfactants chosen among higher alcohols, higher fatty acids, glycerine fatty acid esters, sucrose fatty acid esters, sorbitan fatty acid esters, polyoxyethylenes, sorbitan fatty acid esters and polyglycerine fatty acid esters,
   (l) solubilizing agents consisting of organic acids chosen among fumaric acid, succinic acid and malic acid,
   (m) stabilizers consisting of benzoic acid, sodium benzoate, ethyl parahydroxybenzoate.
[2] The anti-fatigue combination product of [1], wherein the coenzyme Q is coenzyme Q₁₀.
[3] An anti-fatigue combination product consisting of
   (a) reduced coenzyme Q represented by the following formula (1) : 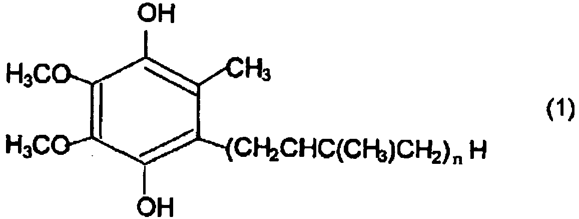 wherein n is an integer of 1 to 12, and oxidized coenzyme Q represented by the following formula (2): 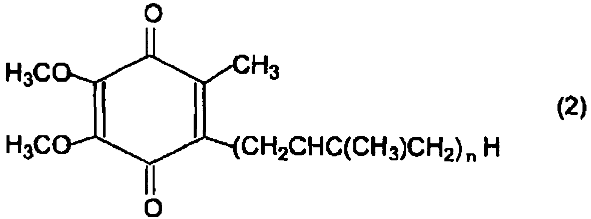 wherein n is an integer of 1 to 12, wherein the proportion of reduced coenzyme Q relative to the entire coenzyme Q is not less than 60 wt%,
   (b) lipoic acid, dihydrolipoic acid, lipoamide, lipoyllysin, di-6,8-bisnorlipoic acid, or tetranorlipoic acid, or a pharmaceutically acceptable salt thereof,
      wherein the weight ratio of reduced coenzyme Q to (b) is within the range of 10:1 to 1:10, and
   (c) optionally at least one antioxidant selected from the group consisting of vitamin C, probucol, lycopene, vitamin A, carotenoids, vitamin B, flavonoids, polyphenols, glutathione, selenium, sodium thiosulfate, vitamin E, pycnogenol, flavangenol, pyrroloquinoline quinone, superoxide dismutase (SOD), glutathione peroxidase, glutathione-S-transferase, glutathione reductase, catalase and ascorbate peroxidase; and optionally one or more carriers chosen from the groups
   (d) to (m) of [1].
[4] The anti-fatigue combination product of [3], wherein the reduced coenzyme Q is reduced coenzyme Q₁₀ and the oxidized coenzyme Q is oxidized coenzyme Q₁₀.
[5] The anti-fatigue combination product of any one of [1] to [4], wherein the lipoic acid is R-lipoic acid.
[6] The anti-fatigue combination product of any one of [1] to [5], wherein the weight of reduced coenzyme Q to (b) is within the range of 5:1 to 1:5.
[7] The anti-fatigue combination product of any one of [1] to [6], wherein said product is a food product.
[8] The combination product of any one of [1] to [6] for use in improving physical fatigue during or after sickness.
[9] A non-therapeutic use of the combination product of any one of [1] to [7], as an anti-fatigue composition.
[10] The non-therapeutic use of [9], wherein the fatigue is physical fatigue due to exercise.
[11] The non-therapeutic use of [9] or [10], wherein the fatigue is a tendency toward easily getting tired because of aging.

### Effect of the Invention

According to the present invention, by using in combination a reduced coenzyme Q and a lipoic acid or a derivative thereof selected from the group consisting of dihydrolipoic acid, lipoamide, lipoyllysin, di-6,8-bisnorlipoic acid, and tetranorlipoic acid, or a pharmaceutically acceptable salt thereof, an unexpected strong anti-fatigue effect can be afforded. In particular, it has been confirmed for the first time in the present invention that even low doses of coenzyme Q and lipoic acid or a derivative thereof as defined above, at which they are ineffective when used singly, express an anti-fatigue effect when used in combination. Conventionally, it is a general understanding that the effects of oxidized coenzyme Q and reduced coenzyme Q on the living body are identical. Therefore, it is surprising that reduced coenzyme Q exhibits such a superior effect. According to the present invention, a safe anti-fatigue pharmaceutical composition or food, which shows assured effects even in a small dose, can be provided.

### Best Mode for Embodying the Invention

Coenzyme Q exists in two forms of oxidized type and reduced type and is known to exist in living bodies, with about 40 to 90% thereof generally occurring in reduced form. The oxidized coenzyme Q can be easily obtained as a commercially available product, or by a conventionally known method such as synthesis, fermentation or extraction from a natural source and the like. The method of preparing reduced coenzyme Q is not particularly limited and for example, a method comprising obtaining coenzyme Q by a conventionally known method such as synthesis, fermentation or extraction from a natural source and concentrating the fraction of reduced coenzyme Q in eluted liquid using chromatography may be employed. In that case, it is also possible to add a common reducing agent such as sodium borohydride, sodium dithionite (sodium hydrosulfite) or ascorbic acid to the above-mentioned coenzyme Q if necessary, and after reducing the oxidized coenzyme Q contained in the above-mentioned coenzyme Q to form reduced coenzyme Q according to a conventional method, subject the same to chromatography for concentration. Reduced coenzyme Q can also be obtained by a method in which the above-mentioned reducing agent is reacted with existing high purity coenzyme Q (oxidized coenzyme Q). Alternatively, microbial cells and the like which contain reduced coenzyme Q may also be used.

In the anti-fatigue composition of the present invention, while reduced coenzyme Q serves as the active and essential ingredient coenzyme Q, a coenzyme Q, which is a mixture of reduced coenzyme Q and oxidized coenzyme Q, may be used as the active ingredient. When a coenzyme Q, which is a mixture of reduced coenzyme Q and oxidized coenzyme Q, is used, the proportion of reduced coenzyme Q relative to the entire coenzyme Q is not less than 60 wt%, preferably not less than 80 wt% and more preferably not less than 95 wt%.

The proportion of the reduced form and the oxidized form in coenzyme Q may be generally determined by a method in which quantities of oxidized coenzyme Q and reduced coenzyme Q in a sample are measured by an HPLC system using a UV detector or the proportion can be calculated from the quantity ratio, or by a method in which the proportion of oxidized coenzyme Q and reduced coenzyme Q is calculated from a peak area according to an HPLC system to which an electrochemical detector is attached. The system with an electrochemical detector is very useful for measuring the proportion of the reduced form existing in living bodies or samples in trace amounts because the system can specifically measure oxidation-reduction substances and has a high sensitivity. Specifically, an electrochemical detector made by Shiseido Co., Ltd. was attached to an HPLC analytical system made by Shimadzu Corporation, and the measurement was conducted under the following HPLC conditions: column: YMC-Pack (ODS-A303), detection wavelength: 275 nm, mobile phase: methanol (88%), hexane (12%), flow rate: 1 ml/min.

The reduced coenzyme Q and the oxidized coenzyme Q that can be used in the present invention are those in which the number of repeating units (n in the formulas) in the side chain is 1 to 12, as represented by the above-mentioned formulas (1) and (2). Of these, those containing 10 repeating units in the side chain, i.e., reduced coenzyme Q₁₀ or oxidized coenzyme Q₁₀, can be suitably used from the viewpoint that sufficient effects can be obtained in human and many pets such as dogs.

The chemical name of lipoic acid is 1,2-dithioran-3-pentanoic acid (thioctic acid). Lipoic acid and lipoic acid derivatives can be produced by a conventionally known method such as synthesis, fermentation, extraction from a natural source, and the like. Moreover, a commercially available product can be used as is. Alternatively, yeast, other fungus, dried product thereof and food that highly contain lipoic acid or the lipoic acid derivatives as defined as (b) in the claims can also be used.

As used herein, a derivative refers to a compound obtained by a small structural modification in a certain compound. A compound in which a hydrogen atom or a specific atomic group is substituted by another atom or another atomic group is understood to be a derivative of the original compound.

Lipoic acid includes, for example, α-lipoic acid (mixture of R-form and S-form), R-lipoic acid, or S-lipoic acid. The lipoic acid derivatives for the present invention are dihydrolipoic acid, lipoamide, lipoyllysin, di-6,8-bisnorlipoic acid, and tetranorlipoic acid. Such lipoic acid derivatives include respective optical isomers of the derivative. Furthermore, lipoic acid derivative includes pharmaceutically acceptable salts of lipoic acid and the above-mentioned derivatives. Those may be used alone or in combination of two or more kinds as the lipoic acid or the lipoic acid derivative in the present invention. Preferred are α-lipoic acid and R-lipoic acid, with more preference given to naturally occurring R-lipoic acid.

Examples of the pharmaceutically acceptable salt of lipoic acid or a derivative thereof include, when the lipoic acid derivative contains a basic moiety, salts with hydrochloric acid, sulfuric acid, methanesulfonic acid, fumaric acid, maleic acid, succinic acid, acetic acid, benzoic acid, oxalic acid, citric acid, tartaric acid, carbonic acid, or phosphoric acid. When lipoic acid and the lipoic acid derivative contain an acidic moiety, examples thereof include alkali metal salts such as sodium salt and potassium salt; alkaline earth metal salts such as calcium salt and magnesium salt; and salts formed with a suitable organic ligand, such as quaternary ammonium salt. Hereinafter, "lipoic acid and lipoic acid derivative" are collectively referred to as a "lipoic acid derivative".

In the anti-fatigue composition of the present invention, the weight ratio of reduced coenzyme Q to lipoic acid, dihydrolipoic acid, lipoamide, lipoyllysin, di-6,8-bisnorlipoic acid, or tetranorlipoic acid, or a pharmaceutically acceptable salt thereof, is within the range 10:1 to 1:10, preferably about 5:1 to about 1:5, and more preferably about 1:1.

As a method for analyzing the lipoic acid derivative, a known analytical method can be used. In the present invention, an electrochemical detector (ECD) was connected to an HPLC system, and the lipoic acid derivative was separated using a Hypersil C18 column (125mm x 4.6 mmi.d, particle size 3µm) and the mobile phase of a phosphate buffer (50 mM KH₂PO₄/H₃PO₄, pH 2.7) and acetonitrile (65:35, v/v). Prior to the HPLC analysis, the analysis sample was suction filtered through a filter with pore diameter of 0.45 µm. In the HPLC analysis, the flow rate of the mobile phase was set to 0.7 ml/min.

The dosage form of the anti-fatigue composition of the present invention is not particularly limited, and it may be an oral preparation or a preparation applied directly to the skin. The oral preparation may be a powder preparation or may be formed into a granular preparation by adding a binder. In addition, such powder or granules may be packed into capsules to prepare a capsule preparation.

The anti-fatigue composition of the present invention may contain at least one antioxidant selected from the group consisting of vitamin C, probucol, lycopene, vitamin A, carotenoids, vitamin B, flavonoids, polyphenols, glutathione, selenium, sodium thiosulfate, vitamin E, pycnogenol, flavangenol, pyrroloquinoline quinone, superoxide dismutase (SOD), glutathione peroxidase, glutathione-S-transferase, glutathione reductase, catalase and ascorbate peroxidase. The above-mentioned antioxidants (including antioxidant enzymes) may be used alone, or in a mixture of two or more kinds thereof.

Further, natural oil, oily higher fatty acid, higher fatty acid monoglyceride, medium-chain triglyceride (MCT), a surfactant or a mixture thereof may be added, and these oily substances may be packed as is to prepare soft capsules. In that case, capsules composed mainly of gelatin or capsules composed mainly of other water soluble polymer substances may be used. In addition, such capsules include microcapsules. Alternatively, the composition may be formed into liquid to prepare a drinkable preparation.

Pharmaceutically acceptable carriers (preparation ingredients) other than the above-mentioned reduced coenzyme Q, lipoic acid derivatives as defined above, and oxidized coenzyme Q or the optional antioxidant as defined above to be added or contained as necessary, may be suitably added to and mixed with the anti-fatigue composition of the present invention by a conventional method.

Therefore, the anti-fatigue composition of the present invention may also optionally contain one or more carriers chosen from the following groups:
- oils consisting of natural oil, oily higher fatty acid, higher fatty acid monoglyceride, medium-chain triglyceride (MCT)or a mixture thereof,
- excipients consisting of saccharose, lactose, glucose, cornstarch, mannitol, crystalline cellulose, calcium phosphate, calcium sulfate,
- disintegrants consisting of starch, agar, calcium citrate, calcium carbonate, sodium hydrogen carbonate, dextrin, crystalline cellulose, carboxymethyl cellulose, tragacanth,
- lubricants consisting of talc, magnesium stearate, polyethylene glycol, silica, hydrogenated vegetable oil,
- binders consisting of ethyl cellulose, methyl cellulose, hydroxypropylmethyl cellulose, tragacanth, shellac, gelatin, gum arabic, polyvinylpyrrolidone, polyvinyl alcohol, polyacrylic acid, polymethacrylic acid, sorbitol,
- colorants,
- anti-agglomeration agents consisting of stearic acid, talc, light anhydrous silicic acid, hydrous silicon dioxide,
- absorption promoters consisting of surfactants chosen among higher alcohols, higher fatty acids, glycerine fatty acid esters, sucrose fatty acid esters, sorbitan fatty acid esters, polyoxyethylenes, sorbitan fatty acid esters and polyglycerine fatty acid esters,
- solubilizing agents consisting of organic acids chosen among fumaric acid, succinic acid and malic acid,
- stabilizers consisting of benzoic acid, sodium benzoate, ethyl parahydroxybenzoate.

The above-mentioned colorant is not particularly limited and those permitted to be added to medicines and food products may be used.

When applying directly to the skin, the dosage form is not particularly limited, and examples of dosage form include those prepared by dissolving, or mixing and dispersing the composition of the present invention in a suitable base, into cream, paste, jelly, gel, emulsion or liquid (ointment, liniment, lotion, cream, spray, etc.); those obtained by dissolving, or mixing and dispersing the composition of the present invention in a suitable base and spreading the resultant on a support (poultices, etc.); and those obtained by dissolving, or mixing and dispersing the composition of the present invention in an adhesive and spreading the resultant on a support (plaster, tape, etc.). As the base and adhesive, those usually used for medicines and cosmetics may be used according to need within the range in which the effect of the present invention is not damaged.

In general, and outside the scope of the invention as claimed, the anti-fatigue composition may also contain other revitalizing ingredients. Suitable examples of revitalizing ingredients include, but not limited to, creatine, taurine, vitamin B₁, vitamin B derivatives, amino acids and the like. These may be used alone, or two or more kinds of these may be mixed. By mixing these ingredients with the coenzyme Q and the lipoic acid derivative in the present invention, further additive or synergistic effects can be expected.

In general, and outside the scope of the invention as claimed, the anti-fatigue composition of the present invention may also contain a supplemental nutrient. Examples of supplemental nutrient include, but not limited to, amino acids, metal ions, saccharides, proteins, fatty acids, vitamins and the like.

When the anti-fatigue composition of the present invention is processed into food, examples of the form thereof include, but are not limited to, edible fat and oil composition, cooking oil, spray oil, butter, margarine, shortening, whipping cream, concentrated milk, whiteners, dressings, pickle liquids, breads, cakes, pies, cookies, Japanese confectionaries, snacks, fried snacks, chocolates and chocolate confectionaries, rice confectionaries, roux, sauce, basting, toppings, ice creams, noodles, bread mix, fried food, processed meat products, fish paste products, frozen food such as frozen entrees, frozen meat and frozen vegetables, rice, jam, cheese, cheese food, cheese-like food, chewing gums, candies, fermented milk, canned food, and drinks.

The present invention also provides the anti-fatigue composition as claimed which is a combination product for use in improving physical fatigue during or after sickness.

The present invention further provides a non-therapeutic use of the combination product as an anti-fatigue composition, and preferably where the fatigue is physical fatigue due to exercise. More preferably, the non-therapeutic use according to the present invention is wherein the fatigue is a tendency toward easily getting tired because of aging.

The "anti-fatigue effect" of the combination product of the present invention as defined above is directed to subjects including human, and other mammals such as dog and cat.

The "effective dose" used herein means an amount containing the active ingredients capable of providing an anti-fatigue effect to the subject. In this case, the anti-fatigue effect may be objective (that is, measurable by some test or marker) or subjective (that is, felt effective by the subject). The dose level and frequency of use of the claimed combination vary depending on the potency of each active ingredient to be used, the metabolic stability and duration of the action of each active ingredient, the species, age, body weight, general health condition, and sex of a subject person (subject animal), diet, mode and time of administration, clearance rate, combination of drugs, severity of the condition to be treated, and the type of treatment the subject person (subject animal) experienced.

The determination of the effective dose is within the ordinary technique of a person skilled in the art, and a person skilled in the art can determine the dose based on his/her experimentation or experience. Examples of the effective dose in the present invention include a combination of about 0.01 mg-1000 mg/kg body weight/day of reduced coenzyme Q and about 0.01-1000 mg/kg body weight/day of lipoic acid derivative as defined as (b) in the claims; preferably about 0.01-500 mg/kg body weight/day of reduced coenzyme Q and about 0.01-500 mg/kg body weight/day of said lipoic acid derivative, and particularly about 0.1-500 mg/kg body weight/day of reduced coenzyme Q and about 0.1-500 mg/kg body weight/day of said lipoic acid derivative. The above-mentioned effective dose can be administered singly or in multiple portions, generally by an oral route. For example, non-oral administration route such as dermal administration, enteric administration and the like can be employed.

The composition of the present invention can also be considered as a synergistic composition. By the term "synergistic composition" used here is meant a composition which exhibits, due to the interaction between the reduced coenzyme Q and the lipoic acid derivative as defined as (b) in the claims, an activity greater than that expected from the action observed when each is separately applied, i.e., an additive activity. Example of the synergistically effective dose of the synergistic composition is a dose smaller than the aforementioned effective doses. For example, about 0.01 mg-300 mg/kg body weight/day of reduced coenzyme Q and about 0.01-300 mg/kg body weight/day of lipoic acid derivative as defined as (b) in the claims, preferably about 0.01-200 mg/kg body weight/day of reduced coenzyme Q and about 0.01-200 mg/kg body weight/day of said lipoic acid derivative, and particularly preferably about 0.1-200 mg/kg body weight/day of reduced coenzyme Q and about 0.1-200 mg/kg body weight/day of said lipoic acid derivative.

The determination of the effective dose of each active ingredient contained in the composition of the present invention is within the ordinary technique of a person skilled in the art, and a person skilled in the art can determine the dose based on his/her experimentation or experience. Examples of the effective dose is 0.01-1000 mg of reduced coenzyme Q in the composition and about 0.01-1000 mg of lipoic acid derivative in the composition as defined as (b) in the claims; preferably about 0.01-500 mg of reduced coenzyme Q in the composition and about 0.01-500 mg of said lipoic acid derivative in the composition, and particularly preferably about 0.1-500 mg of reduced coenzyme Q in the composition and about 0.1-500 mg of said lipoic acid derivative in the composition.

The anti-fatigue composition of the present invention can be produced by a method comprising obtaining reduced coenzyme Q by an existing method and mixing the reduced coenzyme Q and a lipoic acid derivative as defined as (b) in the claims. Alternatively, the oxidized coenzyme Q and said lipoic acid derivative are mixed and, at some time point in the production process, for example, before or after mixing the oxidized coenzyme Q and said lipoic acid, at least a part of the oxidized coenzyme Q may be converted to reduced coenzyme Q.

The form of the product and the method and mode of preservation of the product of the anti-fatigue composition consisting of reduced coenzyme Q and a lipoic acid derivative of the present invention as defined as (b) in the claims can be appropriately determined according to the product design and the purpose of use of the anti-fatigue composition.

### Examples

Hereinafter, the present invention is described in more detail referring to Examples.

The following examples describe preparation of reduced coenzyme Q10, formulations according to the invention comprising reduced coenzyme Q10 and uses of such formulations. Examples outside the scope of the appended claims are to be considered illustrative.

### (Production Example 1) Preparation of reduced coenzyme Q₁₀

To 1000 g of ethanol were added 100 g of oxidized coenzyme Q₁₀ (purity 99.4%) and 60 g of L-ascorbic acid. The mixture was stirred at 78°C to conduct a reduction reaction. After 30 hours, the mixture was cooled to 50°C and with maintaining the temperature, 330 g of ethanol and 70 g of water were added thereto. With stirring the ethanol solution (containing 100 g of reduced coenzyme Q₁₀), the solution was cooled to 2°C at a cooling rate of 10°C/hour to obtain white slurry. The obtained slurry was filtrated under reduced pressure and the resulting wet crystal was washed with cold ethanol, cold water and cold ethanol in that order (temperature of cold solvent used for washing: 2°C), and further, the wet crystal was dried under reduced pressure (20 to 40°C, 1 to 30 mmHg) to obtain 97 g of reduced coenzyme Q₁₀ (containing 2% oxidized coenzyme Q₁₀) white dry crystals. All procedures except drying under reduced pressure were carried out in a nitrogen atmosphere.

### (Production Example 2)

100 g of oxidized coenzyme Q₁₀ was dissolved in 1000 g of a heptane solution at 25°C. With stirring, an aqueous solution obtained by adding 1000 g of water to 100 g of sodium hydrosulfite (purity 75% or higher) as a reducing agent and dissolving the same therein was gradually added to the above-mentioned heptane solution to conduct a reduction reaction while controlling to 25°C, pH 4 to 6. After 2 hours, the aqueous phase was removed from the reaction mixture and the heptane phase was washed with 1000 g of deaerated saturated saline 6 times. The heptane phase was subjected to solvent replacement under reduced pressure and a 7% (w/w) ethanol solution of reduced coenzyme Q₁₀ (containing 100 g of reduced coenzyme Q₁₀) of a temperature of 50°C was prepared. To the ethanol solution was added 50 g of water and with stirring, the solution was cooled to 2°C at a cooling rate of 10°C/hour to allow crystal precipitation. The obtained slurry was filtrated under reduced pressure and the resulting wet crystal was washed with cold ethanol, cold water and cold ethanol in that order (temperature of cold solvent used for washing: 2°C), and further, the wet crystal was dried under reduced pressure (20 to 40°C, 1 to 30 mmHg) to give 97 g of reduced coenzyme Q₁₀ (containing 2% oxidized coenzyme Q₁₀) white dry crystals. All the operations except vacuum drying were performed in a nitrogen atmosphere.

### (Example 1) Treadmill test using young rat

Using Sprague-Dawley male rats (7 to 10 week old, n=20), anti-fatigue effects brought about by reduced coenzyme Q₁₀ alone (about 2% of oxidized coenzyme Q₁₀ included), lipoic acid alone, and combination use of reduced coenzyme Q₁₀ and lipoic acid were evaluated with a treadmill. In the test, rats were run at a speed of 10 m/min using a treadmill apparatus (model NK-73-4 made by Natume Seisakusho Co., Ltd.), and the speed was increased stepwise, by 5 m/min every 3 min, and the time when the rats became unable to run (maximum running time) was measured.

As substances to be tested, 0.5% sodium carboxymethylcellulose (CMC-Na) aqueous solutions of reduced coenzyme Q₁₀ (about 2% oxidized coenzyme Q₁₀ included) or lipoic acid (α-lipoic acid (manufactured by Tokyo Chemical Industry Co., Ltd.)) were each prepared and the solutions were orally administered to rats at a dose of 100 mg/kg for reduced coenzyme Q₁₀ or at a dose of 30 mg/kg for lipoic acid. Rats were divided into four groups of a solvent administered group, reduced coenzyme Q₁₀ only administered group, lipoic acid only administered group, and a reduced coenzyme Q₁₀ and lipoic acid combined group (reduced coenzyme Q₁₀ and lipoic acid were administered almost simultaneously). The maximum running time was measured before and three hours after the administration and an extension of the maximum running time (after the sample administration relative to before the administration) was calculated. A 0.5% CMC-Na aqueous solution was administered to the control group. The maximum running time values are shown in Table 1.

**[Table 1]**

| | Control group | Reduced coenzyme Q₁₀ group | Lipoic acid group | Reduced coenzyme Q₁₀ + lipoic acid |
|---|---|---|---|---|
| Average (second) | -11.7 | 17.5 | 7.3 | 60.9 |
| S.D. | 25.0 | 41.0 | 28.0 | 60.7 |
| Test | | | | P<0.001 |

In the control group, the difference in the maximum running times before and after the administration of the test substance is -11.7 ± 25 seconds, which suggested that the maximum running time slightly decreased. In the lipoic acid or reduced coenzyme Q₁₀ only administered group, no significant extension of the maximum running time of the rats were observed before and after the administration, but in the reduced coenzyme Q₁₀ and lipoic acid administered group, the maximum running times of the rats significantly increased compared to those of the control group. In other words, by combining reduced coenzyme Q₁₀ and lipoic acid, an anti-fatigue effect which was not apparent when they are individually used is now clearly shown, proving that the combination of these substances generates a synergistic anti-fatigue effect.

### (Formulation Example 1)

Olive oil was heated to 60°C and coenzyme Q₁₀ (reduced coenzyme Q₁₀:oxidized coenzyme Q₁₀ = 85:15) and α-lipoic acid melted also at 60°C were added thereto. Vitamin E was gradually added to the mixture until homogeneous and soft capsules were prepared according to a conventional method. Soft capsules containing 20 mg of coenzyme Q₁₀ (17 mg of reduced coenzyme Q₁₀) and 20 mg of α-lipoic acid per capsule were prepared.

| | |
|---|---|
| Coenzyme Q₁₀ | 20 parts by weight |
| (reduced coenzyme Q₁₀:oxidized coenzyme Q₁₀ = 85:15) | |
| α-Lipoic acid | 20 parts by weight |
| Vitamin E | 15 parts by weight |
| Olive oil | 350 parts by weight |

### (Formulation Example 2)

Olive oil was heated to 60°C and coenzyme Q₁₀ (reduced coenzyme Q₁₀:oxidized coenzyme Q₁₀= 85:15) and R-lipoic acid melted also at 60°C were added thereto. Vitamin E was gradually added to the mixture until homogeneous and soft capsules were prepared according to a conventional method. Soft capsules containing 20 mg of coenzyme Q₁₀ (17 mg of reduced coenzyme Q₁₀) and 20 mg of R-lipoic acid per capsule were prepared.

| | |
|---|---|
| Coenzyme Q10 | 20 parts by weight |
| (reduced coenzyme Q10:oxidized coenzyme Q10 = 85:15) | |
| R-Lipoic acid | 20 parts by weight |
| Vitamin E | 15 parts by weight |
| Olive oil | 350 parts by weight |

### (Formulation Example 3)

Coenzyme Q₁₀ (reduced coenzyme Q₁₀:oxidized coenzyme Q₁₀ = 85:15) and α-lipoic acid were added to a suitable amount of acetone and the mixture was then adsorbed on crystalline cellulose (fine powder) followed by drying. The resultant was mixed with cornstarch and formed into powder according to a conventional method.

| | |
|---|---|
| Coenzyme Q₁₀ | 10 parts by weight |
| (reduced coenzyme Q₁₀:oxidized coenzyme Q₁₀ = 85:15) | |
| α-Lipoic acid | 10 parts by weight |
| Crystalline cellulose | 40 parts by weight |
| Cornstarch | 55 parts by weight |

### (Formulation Example 4)

Coenzyme Q₁₀ (reduced coenzyme Q₁₀:oxidized coenzyme Q₁₀ = 85:15) and R-lipoic acid were added to a suitable amount of acetone and the mixture was then adsorbed on crystalline cellulose (fine powder) followed by drying. The resultant was mixed with cornstarch and formed into powder according to a conventional method.

| | |
|---|---|
| Coenzyme Q₁₀ | 10 parts by weight |
| (reduced coenzyme Q₁₀:oxidized coenzyme Q₁₀ = 85:15) | |
| R-Lipoic acid | 10 parts by weight |
| Crystalline cellulose | 40 parts by weight |
| Cornstarch | 55 parts by weight |

### (Formulation Example 5)

Coenzyme Q₁₀ (reduced coenzyme Q₁₀:oxidized coenzyme Q₁₀ = 85:15) and α-lipoic acid were added to a suitable amount of acetone and the mixture was then adsorbed on crystalline cellulose (fine powder) followed by drying. Cornstarch, lactose, carboxymethylcellulose and magnesium stearate were mixed thereto and an aqueous polyvinylpyrrolidone solution was then added thereto as a binder to form granules according to a conventional method. After adding talc thereto as a lubricant and mixing, tablets containing 20 mg of coenzyme Q₁₀ (17 mg of reduced coenzyme Q₁₀) and 20 mg of α-lipoic acid per tablet were prepared.

| | |
|---|---|
| Coenzyme Q₁₀ | 20 parts by weight |
| (reduced coenzyme Q₁₀:oxidized coenzyme Q₁₀ = 85:15) | |
| α-Lipoic acid | 20 parts by weight |
| Cornstarch | 25 parts by weight |
| Lactose | 15 parts by weight |
| Carboxymethylcellulose | 10 parts by weight |
| Crystalline cellulose | 40 parts by weight |
| Polyvinylpyrrolidone | 5 parts by weight |
| Magnesium stearate | 3 parts by weight |
| Talc | 10 parts by weight |

### (Formulation Example 6)

Coenzyme Q₁₀ (reduced coenzyme Q₁₀:oxidized coenzyme Q₁₀= 85:15) and R-lipoic acid were added to a suitable amount of acetone and the mixture was then adsorbed on crystalline cellulose (fine powder) followed by drying. Cornstarch, lactose, carboxymethylcellulose and magnesium stearate were mixed thereto and an aqueous polyvinylpyrrolidone solution was then added thereto as a binder to form granules according to a conventional method. After adding talc thereto as a lubricant and mixing, tablets containing 20 mg of coenzyme Q₁₀ (17 mg of reduced coenzyme Q₁₀) and 20 mg of R-lipoic acid per tablet were prepared.

| | |
|---|---|
| Coenzyme Q₁₀ | 20 parts by weight |

| | |
|---|---|
| (reduced coenzyme Q₁₀:oxidized coenzyme Q₁₀ = 85:15) | |
| R-Lipoic acid | 20 parts by weight |
| Cornstarch | 25 parts by weight |
| Lactose | 15 parts by weight |
| Carboxymethylcellulose | 10 parts by weight |
| Crystalline cellulose | 40 parts by weight |
| Polyvinylpyrrolidone | 5 parts by weight |
| Magnesium stearate | 3 parts by weight |
| Talc | 10 parts by weight |

### (Formulation Example 7)

After granulating the following ingredients according to a conventional method, the resultant was packed in hard gelatin capsules. Capsules containing 20 mg of coenzyme Q₁₀ (17 mg of reduced coenzyme Q₁₀) and 20 mg of α-lipoic acid per capsule were obtained.

| | |
|---|---|
| Coenzyme Q₁₀ | 20 parts by weight |
| (reduced coenzyme Q₁₀:oxidized coenzyme Q₁₀= 85:15) | |
| α-Lipoic acid | 20 parts by weight |
| Crystalline cellulose | 40 parts by weight |
| Cornstarch | 20 parts by weight |
| Lactose | 62 parts by weight |
| Magnesium stearate | 2 parts by weight |
| Polyvinylpyrrolidone | 3 parts by weight |

### (Formulation Example 8)

After granulating the following ingredients according to a conventional method, the resultant was packed in hard gelatin capsules. Capsules containing 20 mg of coenzyme Q₁₀ (17 mg of reduced coenzyme Q₁₀) and 20 mg of R-lipoic acid per capsule were obtained.

| | |
|---|---|
| Coenzyme Q₁₀ | 20 parts by weight |
| (reduced coenzyme Q₁₀:oxidized coenzyme Q₁₀ = 85:15) | |
| R-Lipoic acid | 20 parts by weight |
| Crystalline cellulose | 40 parts by weight |
| Cornstarch | 20 parts by weight |
| Lactose | 62 parts by weight |
| Magnesium stearate | 2 parts by weight |
| Polyvinylpyrrolidone | 3 parts by weight |

### (Preparation Example 1) Health drink

Water was added to coenzyme Q₁₀ (5 g, reduced coenzyme Q₁₀:oxidized coenzyme Q₁₀=85 : 15), α-lipoic acid (5 g), saccharide (150 g), honey (15 g), ascorbic acid (1 g), citric acid (0.3 g), aspartame (2 g), and a suitable amount of flavoring agent to a total amount of 1000 ml. The mixture was sterilized at 95°C for 20 min. Each 100 ml thereof was aseptically filled in a bottle to give a health drink.

### (Preparation Example 2) Margarine

In a 5 L beaker, a hydrogenated cottonseed oil composition (96 parts), coenzyme Q₁₀ (2 parts, reduced coenzyme Q₁₀:oxidized coenzyme Q₁₀ = 85:15) and α-lipoic acid (2 parts) were dissolved by gently stirring with heating at 60-65°C to give a fat and oil composition. The obtained fat and oil composition (83.5 parts) and water (16.5 parts) were emulsified by stirring in an emulsion tank at 60-65°C for 15 min and rapidly cooled to and kneaded at 15°C. The margarine prepared in this manner did not have any problem in flavor and physical properties.

### (Preparation Example 3) Bread

To flour (70 parts) were added yeast (2 parts), yeast food (0.1 part) and then water (40 parts). The mixture was kneaded in a mixer to give an intermediate dough. After 4 hr of preliminary fermentation, flour (30 parts), sugar (5 parts), margarine (6 parts) containing coenzyme Q₁₀ and α-lipoic acid, prepared in Preparation Example 2, table salt (2 parts), skim milk (3 parts) and water (23 parts) were further added to give a final dough. Bread was made in a final fermentation at 38°C for 50 min and baking at 180°C, 35 min. The obtained bread had excellent flavor and color.

## Claims

1. An anti-fatigue combination product consisting of
(a) reduced coenzyme Q represented by the following formula (1) : 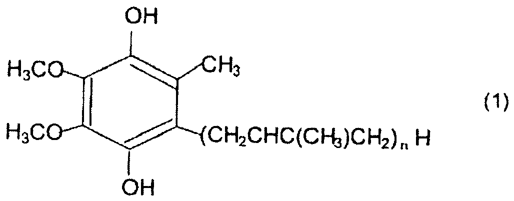 wherein n is an integer of 1 to 12,
(b) lipoic acid, dihydrolipoic acid, lipoamide, lipoyllysin, di-6,8-bisnorlipoic acid, or tetranorlipoic acid, or a pharmaceutically acceptable salt thereof, wherein the weight ratio of (a) to (b) is within the range of 10:1 to 1:10,
(c) optionally at least one antioxidant selected from the group consisting of vitamin C, probucol, lycopene, vitamin A, carotenoids, vitamin B, flavonoids, polyphenols, glutathione, selenium, sodium thiosulfate, vitamin E, pycnogenol, flavangenol, pyrroloquinoline quinone, superoxide dismutase (SOD), glutathione peroxidase, glutathione-S-transferase, glutathione reductase, catalase and ascorbate peroxidase; and
optionally one or more carriers chosen from the following groups:
(d) oils consisting of natural oil, oily higher fatty acid, higher fatty acid monoglyceride, medium-chain triglyceride (MCT)or a mixture thereof,
(e)excipients consisting of saccharose, lactose, glucose, cornstarch, mannitol, crystalline cellulose, calcium phosphate, calcium sulfate,
(f) disintegrants consisting of starch, agar, calcium citrate, calcium carbonate, sodium hydrogen carbonate, dextrin, crystalline cellulose, carboxymethyl cellulose, tragacanth,
(g) lubricants consisting of talc, magnesium stearate, polyethylene glycol, silica, hydrogenated vegetable oil,
(h) binders consisting of ethyl cellulose, methyl cellulose, hydroxypropylmethyl cellulose, tragacanth, shellac, gelatin, gum arabic, polyvinylpyrrolidone, polyvinyl alcohol, polyacrylic acid, polymethacrylic acid, sorbitol,
(i) colorants,
(j) anti-agglomeration agents consisting of stearic acid, talc, light anhydrous silicic acid, hydrous silicon dioxide,
(k) absorption promoters consisting of surfactants chosen among higher alcohols, higher fatty acids, glycerine fatty acid esters, sucrose fatty acid esters, polyoxyethylenes, sorbitan fatty acid esters and polyglycerine fatty acid esters,
(l) solubilizing agents consisting of organic acids chosen among fumaric acid, succinic acid and malic acid,
(m)stabilizers consisting of benzoic acid, sodium benzoate, ethyl parahydroxybenzoate.

2. The anti-fatigue combination product of claim 1, wherein the coenzyme Q is coenzyme Q₁₀.

3. An anti-fatigue combination product consisting of
(a) reduced coenzyme Q represented by the following formula (1) : 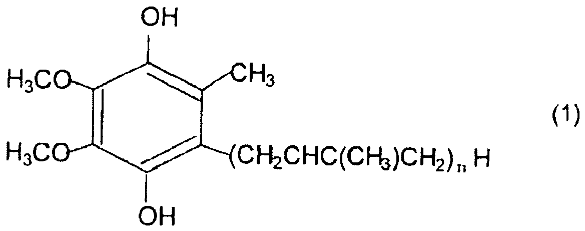 wherein n is an integer of 1 to 12, and oxidized coenzyme Q represented by the following formula (2) : 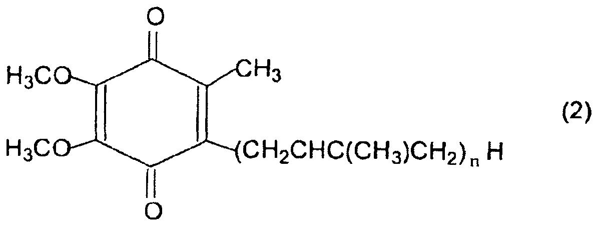 wherein n is an integer of 1 to 12,
wherein the proportion of reduced coenzyme Q relative to the entire coenzyme Q is not less than 60 wt%,
(b) lipoic acid, dihydrolipoic acid, lipoamide, lipoyllysin, di-6,8-bisnorlipoic acid, or tetranorlipoic acid, or a pharmaceutically acceptable salt thereof, wherein the weight ratio of reduced coenzyme Q to (b) is within the range of 10:1 to 1:10, and
(c) optionally at least one antioxidant selected from the group consisting of vitamin C, probucol, lycopene, vitamin A, carotenoids, vitamin B, flavonoids, polyphenols, glutathione, selenium, sodium thiosulfate, vitamin E, pycnogenol, flavangenol, pyrroloquinoline quinone, superoxide dismutase (SOD), glutathione peroxidase, glutathione-S-transferase, glutathione reductase, catalase and ascorbate peroxidase; and
optionally one or more carriers chosen from the groups (d) to (m) as defined in claim 1.

4. The anti-fatigue combination product of claim 3, wherein the reduced coenzyme Q is reduced coenzyme Q₁₀ and the oxidized coenzyme Q is oxidized coenzyme Q₁₀.

5. The anti-fatigue combination product of any one of claims 1 to 4, wherein the lipoic acid is R-lipoic acid.

6. The anti-fatigue combination product of any one of claims 1 to 5, wherein the weight of reduced coenzyme Q to (b) is within the range of 5:1 to 1:5.

7. The anti-fatigue combination product of any one of claims 1 to 6, wherein said product is a food product.

8. The combination product of any one of claims 1 to 6 for use in improving physical fatigue during or after sickness.

9. A non-therapeutic use of the combination product of any one of claims 1 to 7, as an anti-fatigue composition.

10. The non-therapeutic use of claim 9, wherein the fatigue is physical fatigue due to exercise.

11. The non-therapeutic use of claims 9 or 10, wherein the fatigue is a tendency toward easily getting tired because of aging.

## Patentansprüche

1. Kombinationsprodukt gegen Erschöpfung, bestehend aus
(a) reduziertem Coenzym Q, das durch die folgende Formel (1) 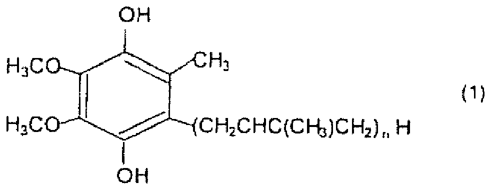 wiedergegeben wird, wobei n eine ganze Zahl von 1 bis 12 ist;
(b) Liponsäure, Dihydroliponsäure, Lipoamid, Lipoyllysin, Di-6,8-bisnor-liponsäure oder Tetranorliponsäure oder einem pharmazeutisch annehmbaren Salz davon, wobei das Gewichtsverhältnis von (a) zu (b) im Bereich von 10:1 bis 1:10 liegt;
(c) gegebenenfalls wenigstens einem Antioxidans, das aus der Gruppe ausgewählt ist, die aus Vitamin C, Probucol, Lycopen, Vitamin A, Carotinoiden, Vitamin B, Flavonoiden, Polyphenolen, Glutathion, Selen, Natriumthiosulfat, Vitamin E, Pycnogenol, Flavangenol, Pyrrolochinolinchinon, Superoxid-Dismutase (SOD), Glutathion-Peroxidase, Glutathion-S-Transferase, Glutathion-Reductase, Katalase und Ascorbat-Peroxidase besteht; und
gegebenenfalls einem oder mehreren Trägern, die aus den folgenden Gruppen ausgewählt sind:
(d) Ölen, die aus natürlichem Öl, einer öligen höheren Fettsäure, einem höhere-Fettsäure-Monoglycerid, mittelkettigem Triglycerid (MCT) oder einem Gemisch davon bestehen;
(e) Arzneimittelhilfsstoffen, die aus Saccharose, Lactose, Glucose, Maisstärke, Mannit, kristalliner Cellulose, Calciumphosphat, Calciumsulfat bestehen;
(f) Sprengmitteln, die aus Stärke, Agar, Calciumcitrat, Calciumcarbonat, Natriumhydrogencarbonat, Dextrin, kristalliner Cellulose, Carboxymethylcellulose, Traganth bestehen;
(g) Gleitmitteln, die aus Talk, Magnesiumstearat, Polyethylenglycol, Siliciumoxid, hydriertem Pflanzenöl bestehen;
(h) Bindemitteln, die aus Ethylcellulose, Methylcellulose, Hydroxypropylmethylcellulose, Traganth, Schellack, Gelatine, Gummi arabicum, Polyvinylpyrrolidon, Polyvinylalkohol, Polyacrylsäure, Polymethacrylsäure, Sorbit bestehen;
(i) Färbemitteln;
(j) Trennmitteln, die aus Stearinsäure, Talk, pyrogener Kieselsäure, Kieselsäure bestehen;
(k) Resorptionsvermittlern, die aus Tensiden bestehen, welche aus höheren Alkoholen, höheren Fettsäuren, Glycerinfettsäureestern, Saccharosefettsäureestern, Polyoxyethylenen, Sorbitanfettsäureestern und Polyglycerinfettsäureestern ausgewählt sind;
(l) Lösungsvermittlern, die aus organischen Säuren bestehen, welche aus Fumarsäure, Bernsteinsäure und Äpfelsäure ausgewählt sind;
(m) Stabilisatoren, die aus Benzoesäure, Natriumbenzoat, Ethylparahydroxybenzoat bestehen.

2. Kombinationsprodukt gegen Erschöpfung gemäß Anspruch 1, wobei es sich bei dem Coenzym Q, um Coenzym Q₁₀ handelt.

3. Kombinationsprodukt gegen Erschöpfung, bestehend aus
(a) reduziertem Coenzym Q, das durch die folgende Formel (1) 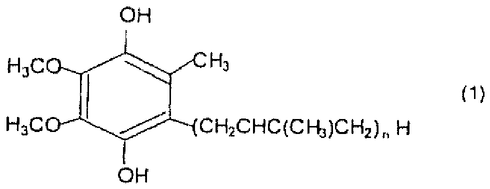 wiedergegeben wird, wobei n eine ganze Zahl von 1 bis 12 ist, und oxidiertem Coenzym Q, das durch die folgende Formel (2) 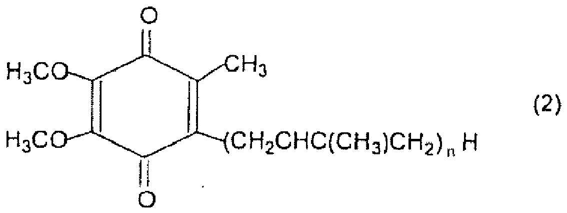 wiedergegeben wird, wobei n eine ganze Zahl von 1 bis 12 ist,
wobei der Anteil an reduziertem Coenzym Q relativ zu dem gesamten Coenzym Q nicht kleiner als 60 Gew.-% ist;
(b) Liponsäure, Dihydroliponsäure, Lipoamid, Lipoyllysin, Di-6,8-bisnor-liponsäure oder Tetranorliponsäure oder einem pharmazeutisch annehmbaren Salz davon, wobei das Gewichtsverhältnis von reduziertem Coenzym Q zu (b) im Bereich von 10:1 bis 1:10 liegt; und
(c) gegebenenfalls wenigstens einem Antioxidans, das aus der Gruppe ausgewählt ist, die aus Vitamin C, Probucol, Lycopen, Vitamin A, Carotinoiden, Vitamin B, Flavonoiden, Polyphenolen, Glutathion, Selen, Natriumthiosulfat, Vitamin E, Pycnogenol, Flavangenol, Pyrrolochinolinchinon, Superoxid-Dismutase (SOD), Glutathion-Peroxidase, Glutathion-S-Transferase, Glutathion-Reductase, Katalase und Ascorbat-Peroxidase besteht; und
gegebenenfalls einem oder mehreren Trägern, die aus den Gruppen (d) bis (m), wie sie in Anspruch 1 definiert sind, ausgewählt sind.

4. Kombinationsprodukt gegen Erschöpfung gemäß Anspruch 3, wobei es sich bei dem reduzierten Coenzym Q um reduziertes Coenzym Q₁₀ und bei dem oxidierten Coenzym Q um oxidiertes Coenzym Q₁₀ handelt.

5. Kombinationsprodukt gegen Erschöpfung gemäß einem der Ansprüche 1 bis 4, wobei es sich bei der Liponsäure um R-Liponsäure handelt.

6. Kombinationsprodukt gegen Erschöpfung gemäß einem der Ansprüche 1 bis 5, wobei das Gewichtsverhältnis von reduziertem Coenzym Q zu (b) im Bereich von 5:1 bis 1:5 liegt.

7. Kombinationsprodukt gegen Erschöpfung gemäß einem der Ansprüche 1 bis 6, wobei das Produkt ein Nahrungsprodukt ist.

8. Kombinationsprodukt gemäß einem der Ansprüche 1 bis 6 zur Verwendung bei der Linderung von körperlicher Erschöpfung während oder nach einer Krankheit.

9. Nichttherapeutische Verwendung des Kombinationsprodukts gemäß einem der Ansprüche 1 bis 7 als Zusammensetzung gegen Erschöpfung.

10. Nichttherapeutische Verwendung gemäß Anspruch 9, wobei es sich bei der Erschöpfung um körperliche Erschöpfung aufgrund von Training handelt.

11. Nichttherapeutische Verwendung gemäß den Ansprüchen 9 oder 10, wobei es sich bei der Erschöpfung um leichte Ermüdbarkeit aufgrund des Alters handelt.

## Revendications

1. Produit combiné anti-fatigue constitué par
(a) un coenzyme Q réduit représenté par la formule (1) suivante : 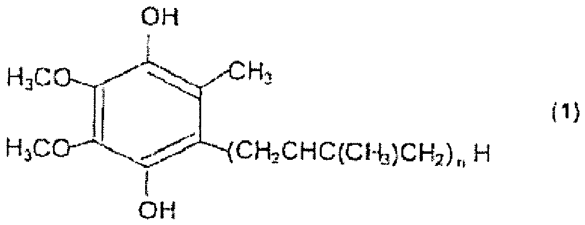 dans laquelle n est un nombre entier de 1 à 12,
(b) l'acide lipoïque, l'acide dihydrolipoïque, le lipoamide, la lipoyllysine, l'acide di-6,8-bisnorlipoïque ou l'acide tétranorlipoïque, ou un sel pharmaceutiquement acceptable de ceux-ci, où le rapport pondéral de (a) sur (b) se situe au sein de la plage de 10:1 à 1:10,
(c) éventuellement au moins un antioxydant choisi dans le groupe constitué par la vitamine C, le probucol, le lycopène, la vitamine A, les caroténoïdes, la vitamine B, les flavonoïdes, les polyphénols, le glutathion, le sélénium, le thiosulfate de sodium, la vitamine E, le pycnogénol, le flavangénol, la pyrroloquinoléine quinone, la superoxyde dismutase (SOD), la glutathion peroxydase, la glutathion-S-transférase, la glutathion réductase, la catalase et l'ascorbate peroxydase ; et
éventuellement un ou plusieurs supports choisis dans les groupes suivants :
(d) des huiles constituées par une huile naturelle, un acide gras supérieur huileux, un monoglycéride d'acide gras supérieur, un triglycéride à chaîne moyenne (MCT) ou un mélange de ceux-ci,
(e) des excipients constitués par le saccharose, le lactose, le glucose, l'amidon de maïs, le mannitol, la cellulose cristalline, le phosphates de calcium, le sulfate de calcium,
(f) des agents de désagrégation constitués par l'amidon, l'agar, le citrate de calcium, le carbonate de calcium, l'hydrogénocarbonate de sodium, la dextrine, la cellulose cristalline, la carboxyméthylcellulose, la gomme adragante,
(g) des lubrifiants constitués par le talc, le stéarate de magnésium, le polyéthylène glycol, la silice, une huile végétale hydrogénée,
(h) des liants constitués par l'éthylcellulose, la méthylcellulose, l'hydroxypropylméthylcellulose, la gomme adragante, la gomme laque, la gélatine, la gomme arabique, la polyvinylpyrrolidone, un polyalcool vinylique, un polyacide acrylique, un polyacide méthacrylique, le sorbitol,
(i) des colorants,
(j) des agents anti-agglomération constitués par l'acide stéarique, le talc, l'acide silicique anhydre léger, le dioxyde de silicium hydraté,
(k) des promoteurs de l'absorption constitués par des surfactants choisis parmi des alcools supérieurs, des acides gras supérieurs, des esters d'acides gras de glycérine, des esters d'acides gras de saccharose, des polyoxyéthylènes, des esters d'acides gras de sorbitane et des esters d'acides gras de polyglycérine,
(l) des agents solubilisants constitués par des acides organiques choisis parmi l'acide fumarique, l'acide succinique et l'acide malique,
(m) des stabilisants constitués par l'acide benzoïque, le benzoate de sodium, le parahydroxybenzoate d'éthyle.

2. Produit combiné anti-fatigue selon la revendication 1, dans lequel le coenzyme Q est le coenzyme Q₁₀.

3. Produit combiné anti-fatigue constitué par
(a) un coenzyme Q réduit représenté par la formule (1) suivante : 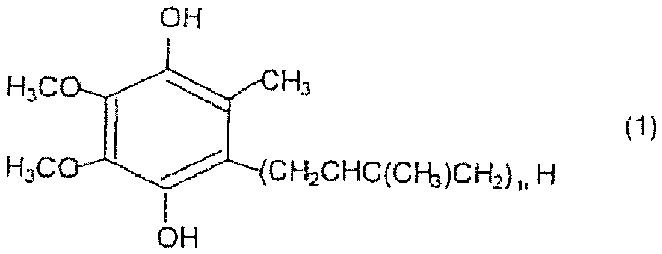 dans laquelle n est un nombre entier de 1 à 12, et un coenzyme Q oxydé représenté par la formule (2) suivante : 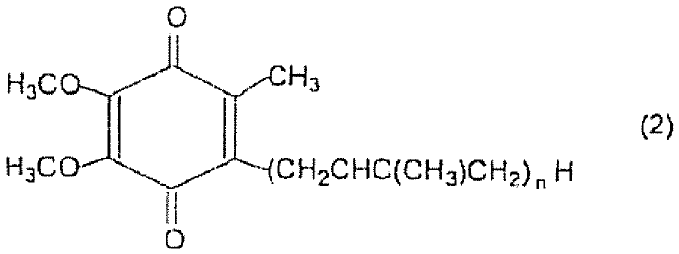 dans laquelle n est un nombre entier de 1 à 12,
où la proportion de coenzyme Q réduit par rapport au coenzyme Q total n'est pas inférieure à 60 % en poids,
(b) l'acide lipoïque, l'acide dihydrolipoïque, le lipoamide, la lipoyllysine, l'acide di-6,8-bisnorlipoïque ou l'acide tétranorlipoïque, ou un sel pharmaceutiquement acceptable de ceux-ci, où le rapport pondéral du coenzyme Q réduit sur (b) se situe au sein de la plage de 10:1 à 1:10, et
(c) éventuellement au moins un antioxydant choisi dans le groupe constitué par la vitamine C, le probucol, le lycopène, la vitamine A, les caroténoïdes, la vitamine B, les flavonoïdes, les polyphénols, le glutathion, le sélénium, le thiosulfate de sodium, la vitamine E, le pycnogénol, le flavangénol, la pyrroloquinoléine quinone, la superoxyde dismutase (SOD), la glutathion peroxydase, la glutathion-S-transférase, la glutathion réductase, la catalase et l'ascorbate peroxydase ; et
éventuellement un ou plusieurs supports choisis dans les groupes (d) à (m) tels que définis dans la revendication 1.

4. Produit combiné anti-fatigue selon la revendication 3, où le coenzyme Q réduit est le coenzyme Q₁₀ réduit et le coenzyme Q oxydé est le coenzyme Q₁₀ oxydé.

5. Produit combiné anti-fatigue selon l'une quelconque des revendications 1 à 4, où l'acide lipoïque est l'acide R-lipoïque.

6. Produit combiné anti-fatigue selon l'une quelconque des revendications 1 à 5, où le poids du coenzyme Q réduit sur (b) se situe au sein de la plage de 5:1 à 1:5.

7. Produit combiné anti-fatigue selon l'une quelconque des revendications 1 à 6, où ledit produit est un produit alimentaire.

8. Produit combiné selon l'une quelconque des revendications 1 à 6, pour une utilisation dans l'amélioration de la fatigue physique durant ou après une maladie.

9. Utilisation non thérapeutique du produit combiné selon l'une quelconque des revendications 1 à 7, en tant que composition anti-fatigue.

10. Utilisation non thérapeutique selon la revendication 9, où la fatigue est une fatigue physique due à l'exercice.

11. Utilisation non thérapeutique selon les revendications 9 ou 10, où la fatigue est une tendance à fatiguer facilement à cause du vieillissement.
